# EUROPÄISCHE PATENTSCHRIFT

(11) **EP 2 178 492 B1**
(45) Veröffentlichungstag und Bekanntmachung des Hinweises auf die Patenterteilung: **14.12.2016**
(21) Anmeldenummer: 08735763.8
(22) Anmeldetag: 03.04.2008
(51) Int. Cl.: A61K 8/41, A61K 8/44, A61Q 5/10

(54) **HAARBEHANDLUNGSMITTEL ENTHALTEND EIN ACYLAMINOSÄUREDERIVAT, EIN HYDROXYALKYLAMIN UND 2-(2-HYDROXYETHYL)-P-PHENYLENDIAMIN**
HAIR TREATMENT AGENT COMPRISING AN ACYLAMINO ACID DERIVATIVE, A HYDROXYALKYLAMINE, AND 2-(2-HYDROXYETHYL)-P-PHENYLENEDIAMINE
AGENTS DE TRAITEMENT CAPILLAIRES CONTENANT UN DÉRIVÉ D'ACIDE ACYLAMINÉ, UNE HYDROXYALKYLAMINE ET UNE 2-(2-HYDROXYÉTHYLE)-P-PHÉNYLÈNEDIAMINE

(30) Priorität: 20.08.2007 DE 102007039330
(43) Veröffentlichungstag der Anmeldung: 28.04.2010
(73) Patentinhaber: Henkel AG & Co. KGaA, 40589 Düsseldorf (DE)
(72) Erfinder: DÖRING, Thomas, AZ 35255 Scottsdale (US); SÜNGER, Georg, 40547 Düsseldorf (DE)
(86) Internationale Anmeldenummer: PCT/EP2008/054017
(87) Internationale Veröffentlichungsnummer: WO 2009/024362

(56) Entgegenhaltungen:
- EP-A- 0 686 389
- WO-A-96/19188
- WO-A-99/37278
- WO-A-2006/097167
- DE-A1- 19 527 121
- US-A1- 2002 040 509

## Beschreibung

Die Erfindung betrifft Mittel, welche mindestens ein N-Acylaminosäurederivat, 2-(2-Hydroxyethyl)-p-phenylendiamin und mindestens ein Hydroxyalkylamin enthalten. Diese Mittel werden zur Verwendung als Färbemittel von keratinhaltigen Fasern, insbesondere unter Nutzung eines Verfahrens zur Behandlung keratinhaltiger Fasern eingesetzt.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.
Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 4-Amino-3-methylphenol, 2-Aminomethyl-4-aminophenol, 4,5-Diamino-1-(2-hydroxyethyl)pyrazol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diamino-propan-2-ol.

Als Kupplerkomponenten werden in der Regel m-Phenylendiaminderivate, Naphthole, Pyridinderivate, Resorcin und Resorcinderivate, Pyrazolone und m-Aminophenole verwendet. Als Kupplersubstanzen eignen sich insbesondere 1-Naphthol, 1,5-Dihydroxynaphthalin, 2,7-Dihydroxynaphthalin, 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazol-5-on, 2,4-Dichlor-3-aminophenol, 1,3-Bis-(2,4-diaminophenoxy)-propan, 2-Amino-3-hydroxypyridin, 4-Chlorresorcin, 2-Chlor-6-methyl-3-aminophenol, 2-Methylresorcin, 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine weitere Möglichkeit zur Farbveränderung bietet die Verwendung von Färbemitteln, die eine Kombination aus Komponente
A Verbindungen, die eine reaktive Carbonylgruppe enthalten mit Komponente
B Verbindungen, ausgewählt aus (a) CH-aciden Verbindungen und (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten. Die entsprechende Färbemethode (im folgenden Oxofärbung genannt) wird beispielsweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit lässt sich die Methode der Oxofärbung ohne weiteres mit dem oxidativen Färbesystem kombinieren.

Zur Einstellung des pH-Wertes bzw. zur Verbesserung des Farbaufzuges setzt der Fachmann meist Ammoniak ein. Allerdings führt der Einsatz von Ammoniak zu einem unerwünschten Geruch während der Anwendung. Des Weiteren wirkt sich die Gegenwart von Ammoniak nachteilig auf die Haarstruktur aus. Schließlich kommt es durch den Einsatz von Ammoniak zu einer Störung der Hautbarriere, wodurch die Penetration von Färbemitteln in die Haut erleichtert wird, wodurch wiederum potentiell das Risiko einer allergischen Reaktion erhöht wird. Somit geht der Fachmann seit einiger Zeit dazu über, ammoniakarme Färbemittel bereitzustellen.
Zwar besitzen ammoniakarme Färbemittel nicht die vorgenannten Nachteile, jedoch büßen sie an Färbekraft und Farbechtheit ein. Daher ist es ein allgemeines Bestreben, die mit den bekannten Methoden erzielbaren Färbungen möglichst ohne Ammoniak durchzuführen und in Ihrem Farbergebnis zu intensivieren. Eine hohe Färbekraft trägt zur Wirtschaftlichkeit von Färbemitteln bei. Außerdem sollen die erzielten Färbungen ein hohes Maß an Farbechtheit z.B. gegen Schweiß, Waschen, Licht oder Reibung aufweisen und müssen insbesondere im Rahmen der Haarpflege kompatibel mit anderen Haarbehandlungsmitteln sein. Schließlich soll der Verzicht auf Ammoniak Hautirritationen vorbeugen und Haarcoloration ohne Allergierisiko erlauben.
EP 0 686 389 A1 betrifft oxidative Färbemittel enthaltend die Kombinationen des Entwicklers 2-(2-Hvdroxyethyl)-p-phenylendiamin mit den Kupplern 2-Methylresorcin und 3-Aminophenol.
Es wurde überraschend gefunden, dass durch Verwendung von 2-(2-Hydroxyethyl)-p-phenylendiamin, mindestens eines Vertreters aus der Gruppe der N-Acylaminosäurederivate sowie mindestens eines Hydroxyalkylamins in Färbemitteln die Intensität von Haarfärbungen erhöht werden kann, und dass durch Verwendung von 2-(2-Hydroxyethyl)-p-phenylendiamin, mindestens eines Vertreters aus der Gruppe der N-Acylaminosäurederivate sowie mindestens eines Hydroxyalkylamins in Färbemitteln gleichzeitig die Haut weniger gereizt und durch verminderte Hautbarrierestörung das Allergierisiko reduziert werden kann.

Die erzielte Farbintensivierung bietet einen generellen Vorteil für alle Färbemittel, ist jedoch insbesondere für ammoniakfreie Färbemittel notwendig. Durch Absenkung der eingesetzten Farbstoffvorprodukte kann wiederum das Allergierisiko gesenkt werden.

Ein erster Gegenstand der Erfindung ist daher ein Mittel zur Färbung keratinhaltiger Fasern, insbesondere menschlicher Haare, welches
(i) 2-(2-Hydroxyethyl)-p-phenylendiamin und/oder mindestens eines seiner physiologisch verträglichen Salze,
(ii) mindestens eine Verbindung aus den N-acylierten Aminosäurederivaten der Formel (I) 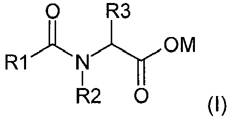 worin
   R¹ eine (C₇ bis C₃₀)-Alkylgruppe, (C₇ bis C₃₀)-Alkenylgruppe oder eine
   (C₇ bis C₃₀)-Hydroxyalkylgruppe bedeutet,
   R² und R³ voneinander unabhängig für ein Wasserstoffatom, eine (C₁ bis C₄)-Alkylgruppe oder eine (C₁ bis C₄)-Hydroxyalkylgruppe steht,
   und
   M ein Wasserstoffatom oder ein Äquivalent eines ein- oder mehrwertigen Kations bedeutet,
(iii) sowie mindestens ein Hydroxyalkylamin
enthält.

Die gemäß Formel (I) erfindungsgemäßen (C₇ bis C₃₀)-Alkylgruppen können linear oder verzweigt sein. Die gemäß Formel (I) erfindungsgemäßen (C₇ bis C₃₀)-Alkenylgruppen können linear oder verzweigt sein sowie können eine oder mehrere Doppelbindungseinheiten unterschiedlicher Isomerie (E- bzw. Z-Isomer) enthalten. Die erfindungsgemäßen (C₇ bis C₃₀)-Hydroxyalkylgruppen können linear oder verzweigt sein und müssen mindestens eine freie Hydroxy-gruppe enthalten. Der Rest R¹ der Formel (I) steht bevorzugt für eine (C₉ bis C₂₃)-Alkylgruppe, (C₉ bis C₂₃)-Alkenylgruppe oder eine (C₉ bis C₂₃)-Hydroxyalkylgruppe, besonders bevorzugt für einen Rest, ausgewählt aus der Liste, die gebildet wird aus Nonyl, Undecyl, Tridecyl, Pentadecyl, Heptadecyl, Nonadecyl, Henicosanyl, 15-Methylhexadecyl, Heptadec-8-enyl, Heptadeca-8,11-dienyl, Nonadeca-4,7,10,13-tetraenyl, Heptadeca-8,11,14-trienyl und 11-Hydroxyheptadecyl.

Die Reste R² und R³ gemäß Formel (I) stehen bevorzugt unabhängig voneinander für einen Rest, ausgewählt aus der Gruppe, die gebildet wird aus Wasserstoffatom, Methyl, Ethyl, Isopropyl, n-Propyl und 2-Hydroxyethyl. Besonders bevorzugt stehen der besagte Rest R² und der besagte Rest R³ der Formel (I) unabhängig voneinander für ein Wasserstoffatom oder eine Methylgruppe. Wenn die Verbindung der Formel (I) als Säure vorliegt, bedeutet der Rest M ein Wasserstoffatom. Wenn die Verbindung der Formel (I) als Salz vorliegen, stehen M für ein Äquivalent eines ein- oder mehrwertigen Kations. Das ein oder mehrwertige Kation M^{z+} mit jeweils einer Ladungszahl z von eins oder höher dient lediglich aus Gründen der Elektroneutralität zur Kompensation der einfach negativen Ladung des bei Salzbildung vorliegenden Carboxylatfragments -COO⁽⁻⁾ der Formel (I). Das dafür zu verwendende Äquivalent des entsprechenden Kations beträgt 1/z. Das Fragment - COOM der Formel (I) steht im Fall der Salzbildung für die Gruppe:

-COO⁽⁻⁾ 1/z (M^{z+})

Als ein- oder mehrwertige Kationen M^{z+} kommen prinzipiell alle physiologisch verträglichen Kationen in Frage. Insbesondere sind dies Metallkationen der physiologisch verträglichen Metalle aus den Gruppen la, Ib, IIa, IIb, IIIb, VIa oder VIII des Periodensystems der Elemente sowie kationische organische Verbindungen mit quaterniertem Stickstoffatom. Letztere werden beispielsweise durch Protonierung primärer, sekundärer oder tertiärer organischer Amine mit einer Säure, wie z.B. mit einer Verbindung der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht in der Formel (I) bevorzugt für ein Wasserstoffatom, ein Alkalimetallion, für ein halbes Äquivalent eines Erdalkalimetallions oder ein halbes Äquivalent eines Zinkions, besonders bevorzugt für ein Wasserstoffatom, ein Natriumion, ein Kaliumion, ½ Kalziumion, ½ Magnesiumion oder ½ Zinkion.

Die Verbindungen der Formel (I) werden bevorzugt ausgewählt aus mindestens einem Vertreter aus der Gruppe, die gebildet wird,
aus N-Lauroylsarcosin, N-Myristoylsarcosin, N-Palmitoylsarcosin, N-Oleylsarcosin, N-Cocoylsarcosin (wobei hier eine Gruppe von Verbindungen vorliegt und Cocoyl der Zusammensetzung des Fettsäureschnitt des Kokosnussöls entspricht), N-Palmkernsarcosin (wobei hier eine Gruppe von Verbindungen vorliegt und Palmkern der Zusammensetzung des Fettsäureschnitt des Palmkernöls entspricht),
N-Lauroylglycin, N-Myristoylglycin, N-Palmitoylglycin, N-Oleylglycin, N-Cocoylglycin (wobei hier eine Gruppe von Verbindungen vorliegt und Cocoyl der Zusammensetzung des Fettsäureschnitt des Kokosnussöls entspricht), N-Palmkernglycin (wobei hier eine Gruppe von Verbindungen vorliegt und Palmkern der Zusammensetzung des Fettsäureschnitt des Palmkernöls entspricht), N-Lauroylalanin, N-Myristoylalanin, N-Palmitoylalanin, N-Oleylalanin, N-Cocoylalanin (wobei hier eine Gruppe von Verbindungen vorliegt und Cocoyl der Zusammensetzung des Fettsäureschnitt des Kokosnussöls entspricht) und N-Palmkernalanin (wobei hier eine Gruppe von Verbindungen vorliegt und Palmkern der Zusammensetzung des Fettsäureschnitt des Palmkernöls entspricht), sowie den physiologisch verträglichen Salzen der vorgenannten N-Acylaminosäurederivate.

Die Triglyceride des Kokosöls weisen folgende Fettsäurezusammensetzung auf:

| | |
|---|---|
| 45 bis 51 % | Laurinsäure |
| 16 bis 19 % | Tetradecansäure |
| 8 bis 10 % | Ölsäure |
| 9 bis 11 % | Palmitinsäure |
| 6 bis 9 % | Decansäure |
| 5 bis 8 % | Octansäure |

Die Triglyceride des Palmkernöls weisen folgende Fettsäurezusammensetzung auf:

| | |
|---|---|
| 47 bis 52 % | Laurinsäure |
| 16 bis 19 % | Tetradecansäure |
| 10 bis 18 % | Ölsäure |
| 6 bis 9 % | Palmitinsäure |
| 2 bis 3 % | Stearinsäure |
| 1 bis 3 % | Linolsäure |
| 2 bis 5 % | Decansäure |
| 1 bis 3 % | Octansäure |

Die erfindungsgemäßen Hydroxyalkylamine werden bevorzugt ausgewählt aus primären Aminen mit einem C₂-C₆-Alkylgrundkörper, der mindestens eine Hydroxylgruppe trägt. Besonders bevorzugte Hydroxyalkylamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Hydroxyalkylamine werden ausgewählt aus der Gruppe 2-Aminoethan-1-ol, 2-Amino-2-methylpropan-1-ol und 2-Amino-2-methyl-propan-1,3-diol.

Die erfindungsgemäßen Mittel enthalten 2-(2-Hydroxyethyl)-p-phenylendiamin oder eines seiner physiologisch verträglichen Salze bevorzugt in einer Menge von 0,001 bis 6,0 Gew.-%, besonders bevorzugt von 0,005 bis 5,0 Gew.-%, ganz besonders bevorzugt von 0,01 bis 3,0 Gew.-% jeweils bezogen auf das Gewicht des Färbemittels.

Die erfindungsgemäßen Mittel enthalten mindestens ein Hydroxylamin in einer Menge von 1,0 bis 8,0 Gew.-%, besonders bevorzugt von 1,5 bis 7,5 Gew.-%, ganz besonders bevorzugt von 2,0 bis 7,0 Gew.-% jeweils bezogen auf das Gewicht des Färbemittels.

Die erfindungsgemäßen Mittel enthalten mindestens ein N-Acylaminosäurederivat der Formel (I) bevorzugt in einer Menge von 0,1 bis 10 Gew.-%, besonders bevorzugt von 0,3 bis 8,0 Gew.-%, ganz besonders bevorzugt von 0,5 bis 6,0 Gew.-% jeweils bezogen auf das Gewicht des Färbemittels.

In einer bevorzugten Ausführungsform ist das verwendete Mittel insbesondere ammoniakfrei.

In einer bevorzugten Ausführungsform enthält das verwendete, insbesondere ammoniakfreie Mittel zusätzlich 0 bis 0,5 Gew.-%, insbesondere 0 bis 0,3 Gew.-%, jeweils bezogen auf das Gewicht des Färbemittels, mindestens eines anionischen Tensids, ausgewählt aus
- linearen oder verzweigten C₈ bis C₄₀-Alkylsulfaten,
- linearen oder verzweigten C₈ bis C₄₀-Alkylethersulfaten mit mindestens einer Ethylenoxid-Einheit oder Propylenoxid-Einheit im Molekül.

Die erfindungsgemäßen Mittel enthalten in einer weiteren Ausführungsform zusätzlich mindestens eine weitere farbgebende Komponente, bevorzugt in einer Menge von 0,001 bis 1,0 Gew.-%, besonders bevorzugt von 0,01 bis 1,0 Gew.-%, ganz besonders bevorzugt von 0,1 bis 0,9 Gew.-% jeweils bezogen auf das Gewicht des Färbemittels.

Die farbgebende Komponente wird bevorzugt ausgewählt
(1) aus mindestens einem Oxidationsfarbstoffvorprodukt vom Typ der Entwicklerkomponenten und gegebenenfalls zusätzlich mindestens einer Kupplerkomponente und/oder
(2) aus Oxofarbstoffvorprodukten
   und/oder
(3) aus mindestens einem direktziehenden Farbstoff
   und/oder
(4) aus mindestens einer Vorstufe naturanaloger Farbstoffe.

Es kann erfindungsgemäß bevorzugt sein, die erfindungsgemäßen Entwicklerkomponenten aus der Gruppe auszuwählen, die gebildet wird, aus p-Phenylendiaminderivaten, zweikernigen Entwicklerkomponenten, p-Aminophenol und seinen Derivaten, Pyrimidinderivaten, Pyrazolderivaten sowie Pyrazozlopyrimidinderivaten und den physiologisch verträglichen Salzen dieser Verbindungen. Im folgenden werden erfindungsgemäß bevorzugte Entwicklerkomponenten genannt.

Besonders bevorzugte Entwicklerkomponenten sind p-Phenylendiaminderivate der Formel (E1) 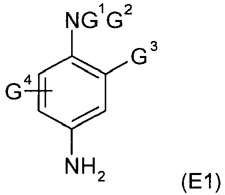 wobei
- G¹ steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen 4'-Aminophenylrest oder einen (C₁ bis C₄)-Alkylrest, der mit einer stickstoffhaltigen Gruppe, einem Phenyl- oder einem 4'-Aminophenylrest substituiert ist; - G² steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest oder einen (C₁ bis C₄)-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴ steht für ein Wasserstoffatom, ein Halogenatom oder einen (C₁ bis C₄)-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden unter der Vorraussetzung, dass es sich bei dem p-Phenylendiaminderivat der Formel (E1) nicht um 2-(2-Hydroxyethyl)-p-phenylendiamin handelt.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1 H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.
Für eine im Rahmen der Erfindung optimale Abdeckung grauer keratinhaltiger Fasern ist es besonders bevorzugt, dem erfindungsgemäßen Mittel zusätzlich p-Toluylendiamin und/oder dessen physiologisch verträglichen Salze in einer Menge von 0,01 bis 0,99 Gew.-%, bezogen auf das Gewicht des Färbemittels, zuzusetzen. Bevorzugt ist wiederum ein Gehalt an p-Toluylendiamin von 0,05 bis 0,6 Gew.-%, insbesondere von 0,15 bis 0,5 Gew.-%, jeweils bezogen auf das Gewicht des Färbemittels.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 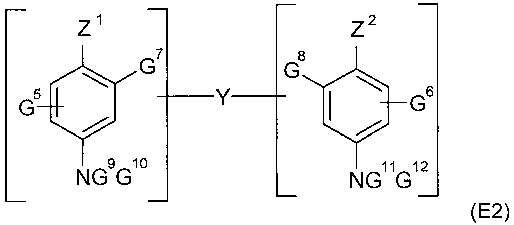 wobei:
- Z¹ und Z² stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶ stehen unabhängig voneinander für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 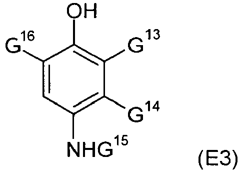 wobei:
- G¹³ steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴ steht für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵ steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Hierbei sind Pyrimidinderivate als heterozyklische Entwicklerkomponenten besonders bevorzugt.

Bevorzugte Pyrimidin-Derivate werden erfindungsgemäß ausgewählt aus Verbindungen gemäß Formel (E4) bzw. deren physiologisch verträglichen Salzen, 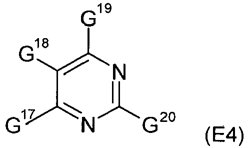 worin
- G¹⁷, G¹⁸ und G¹⁹ unabhängig voneinander für ein Wasserstoffatom, eine Hydroxygruppe, eine (C₁ bis C₄)-Alkoxygruppe oder eine Aminogruppe steht und
- G²⁰ für eine Hydroxygruppe oder eine Gruppe -NG²¹G²² steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷, G¹⁸, G¹⁹ und G²⁰ eine Hydroxygruppe bedeuten und höchstens zwei der Reste G¹⁷, G¹⁸ und G¹⁹ für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²² stehen und höchstens zwei Gruppen aus G¹⁷, G¹⁸, G¹⁹ und G²⁰ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 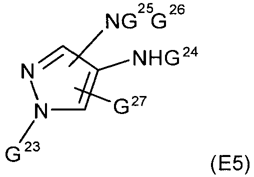 worin
- G²³, G²⁴, G²⁵ stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁶ neben den vorgenannten Gruppen zusätzlich für eine Gruppe -NH₂ stehen kann,
- G²⁶ steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷ steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²⁶ an die 5 Position und der Rest G²⁷ an die 3 Position des Pyrazolzyklus.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Bevorzugte Pyrazolopyrimidin-Derivate sind insbesondere die Derivate des Pyrazolo[1,5-a]pyrimidin der folgenden Formel (E6) und dessen tautomeren Formen, sofern ein tautomeres Gleichgewicht besteht: 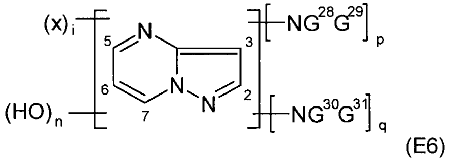 wobei:
- G²⁸, G²⁹ und G³⁰, G³¹ unabhängig voneinander stehen für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest einen (C₁ bis C₄)-Alkoxy-(C₁ bis C₄)-alkylrest, einen (C₁ bis C₄)-Aminoalkylrest, der gegebenenfalls durch ein Acetyl-Ureid- oder einen Sulfonyl-Rest geschützt sein kann, einen (C₁ bis C₄)-Alkylamino-(C₁ bis C₄)-alkylrest, einen Di-[(C₁ bis C₄)-alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Monohydroxyalkyl- oder einen Di[(C₁ bis C₄)-Hydroxyalkyl]-(C₁ bis C₄)-aminoalkylrest,
- die X-Reste stehen unabhängig voneinander für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest, einen (C₁ bis C₄)-Aminoalkylrest, einen (C₁ bis C₄)-Alkylamino-(C₁ bis C₄)-alkylrest, einen Di-[(C₁ bis C₄)alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Hydroxyalkyl- oder einen Di-[(C₁ bis C₄)-hydroxyalkyl]amino-(C₁ bis C₄)-alkylrest, einen Aminorest, einen (C₁ bis C₄)-Alkyl- oder Di-[(C₁ bis C₄)-hydroxyalkyl]aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG²⁸G²⁹ und NG³⁰G³¹ belegen die Positionen (2,3); (5,6); (6,7); (3,5) oder (3,7);
- wenn p + q gleich 1 ist, n den Wert 1 hat, und die Gruppen NG²⁸G²⁹ (oder NG³⁰G³¹) und die Gruppe OH belegen die Positionen (2,3); (5,6); (6,7); (3,5) oder (3,7);

Die in Formel (E7) verwendeten Substituenten sind erfindungsgemäß analog zu den obigen Ausführungen definiert.

Wenn das Pyrazolo[1,5-a]pyrimidin der obenstehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

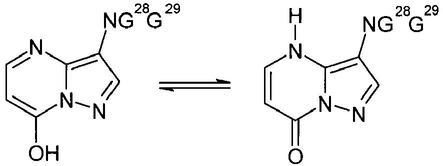

Unter den Pyrazolo[1,5-a]pyrimidinen der oben stehenden Formel (E7) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E6) können wie in der Literatur beschrieben durch Zyklisierung ausgehend von einem Aminopyrazol oder von Hydrazin hergestellt werden.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E6) genannten Reste aufgezählt: Beispiele für (C₁ bis C₄)-Alkylreste sind die Gruppen -CH₃,-CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃,-OC(CH₃)₃, insbesondere eine Methoxy- oder eine Ethoxygruppe. Weiterhin können als bevorzugte Beispiele für eine (C₁ bis C₄)-Monohydroxyalkylgruppe -CH₂OH, -CH₂CH₂OH, -CH₂CH₂CH₂OH, -CHCH(OH)CH₃, -CH₂CH₂CH₂CH₂OH, wobei die Gruppe-CH₂CH₂OH bevorzugt ist.
Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.

Beispiele für (C₁ bis C₄)-Monoalkylaminogruppen sind -NHCH₃, -NHCH₂CH₃, -NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Trialkylammoniumgruppen sind -N⁺(CH₃)₃, -N⁺(CH₃)₂(CH₂CH₃), -N⁺(CH₃)(CH₂CH₃)₂.
Beispiele für (C₁ bis C₄)-Hydroxyalkylaminoreste sind -NH-CH₂CH₂OH, -NH-CH₂CH₂OH, -NH-CH₂CH₂CH₂OH, -NH-CH₂CH₂CH₂OH.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).
Beispiele für Hydroxy-(C₁ bis C₄)-alkoxyreste sind -O-CH₂OH, -O-CH₂CH₂OH, -O-CH₂CH₂CH₂OH, -O-CHCH(OH)CH₃, -O-CH₂CH₂CH₂CH₂OH.
Beispiele für (C₁ bis C₄)-Acetylaminoalkoxyreste sind -O-CH₂NHC(O)CH₃, -O-CH₂CH₂NHC(O)CH₃, -O-CH₂CH₂CH₂NHC(O)CH₃, -O-CH₂CH(NHC(O)CH₃)CH₃, -O-CH₂CH₂CH₂CH₂NHC(O)CH₃.
Beispiele für (C₁ bis C₄)-Carbamoylaminoalkoxyreste sind -O-CH₂CH₂-NH-C(O)-NH₂, -O-CH₂CH₂CH₂-NH-C(O)-NH₂, -O-CH₂CH₂CH₂CH₂-NH-C(O)-NH₂.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Beispiele für (C₁ bis C₄)-Cyanoalkylreste sind -CH₂CN, -CH₂CH₂CN, -CH₂CH₂CH₂CN.
Beispiele für (C₁ bis C₄)-Hydroxyalkylamino-(C₁ bis C₄)-alkylreste sind -CH₂CH₂NH-CH₂CH₂OH, -CH₂CH₂CH₂NH-CH₂CH₂OH, -CH₂CH₂NH-CH₂CH₂CH₂OH, -CH₂CH₂CH₂NH-CH₂CH₂CH₂OH.
Beispiele für Di[(C₁ bis C₄)-Hydroxyalkyl]amino-(C₁ bis C₄)-alkylreste sind -CH₂CH₂N(CH₂CH₂OH)₂, -CH₂CH₂CH₂N(CH₂CH₂OH)₂, -CH₂CH₂N(CH₂CH₂CH₂OH)₂, -CH₂CH₂CH₂N(CH₂CH₂CH₂OH)₂.
Ein Beispiel für Arylgruppen ist die Phenylgruppe.
Beispiele für Aryl-(C₁ bis C₄)-alkylgruppen sind die Benzylgruppe und die 2-Phenylethylgruppe.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,
Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1), 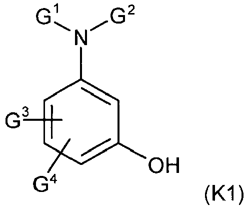 worin
- G¹ und G²: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₂ bis C₄)-Perfluoracylgruppe, eine Aryl-(C₁ bis C₆)-alkylgruppe, eine Amino-(C₁ bis C₆)-alkylgruppe, eine (C₁ bis C₆)-Dialkylamino-(C₁ bis C₆)-alkylgruppe oder eine (C₁ bis C₆)-Alkoxy-(C₁ bis C₆)-alkylgruppe, wobei G¹ und G² gemeinsam mit dem Stickstoffatom einen fünfgliedrigen, sechsgliedrigen oder siebengliedrigen Ring bilden können,
- G³ und G⁴: unabhängig voneinander stehen für ein Wasserstoffatom, ein Halogenatom, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe, eine (C₁ bis C₆)-Alkyox-(C₂ bis C₆)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 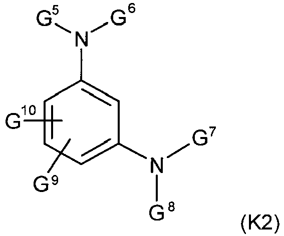 worin
- G⁵, G⁶, G⁷ und G⁸: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G⁹ und G¹⁰: unabhängig voneinander stehen für ein Wasserstoffatom, ein Halogenatom, eine (C₁ bis C₄)-Alkylgruppe, eine ω-(2,4-Diaminophenyl)-(C₁ bis C₄)-alkylgruppe, eine ω-(2,4-Diaminophenyloxy)-(C₁ bis C₄)-alkoxygruppe, eine (C₁ bis C₄)-Alkoxygruppe, eine Hydroxygruppe, eine (C₁ bis C₄)-Alkoxy-(C₂ bis C₄)-alkoxygruppe, eine Arylgruppe, eine Heteroarylgruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 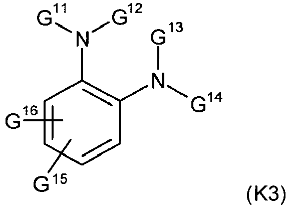 worin
- G¹¹, G¹², G¹³ und G¹⁴: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G¹⁵ und G¹⁶: unabhängig voneinander stehen für ein Wasserstoffatom, ein Halogenatom, eine Carboxylgruppe, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 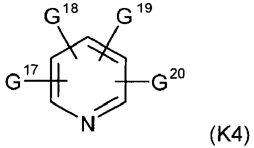 worin
- G¹⁷ und G¹⁸: stehen unabhängig voneinander für eine Hydroxygruppe oder eine Gruppe -NG²¹G²², worin G²¹ und G²² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine Arylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe,
- G¹⁹ und G²⁰: stehen unabhängig voneinander für ein Wasserstoffatom, ein Halogenatom, eine (C₁ bis C₄)-Alkylgruppe oder eine (C₁ bis C₄)-Alkoxygruppe.

Es ist bevorzugt, wenn gemäß Formel (K4) die Reste G¹⁷ und G¹⁸ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 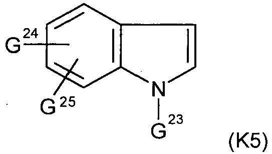 worin
- G²³: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁴: steht für eine Hydroxygruppe oder eine Gruppe -NG²⁶G²⁷, worin G²⁶ und G²⁷ unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G²⁵: Wasserstoffatom, ein Halogenatom oder eine (C₁ bis C₄)-Alkylgruppe,
mit der Maßgabe, dass G²⁴ in meta-Position oder ortho-Position zum Strukturfragment NG²³ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 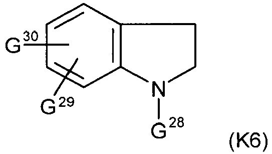 worin
- G²⁸: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁹: steht für eine Hydroxygruppe oder eine Gruppe -NG³¹G³², worin G³¹ und G³² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G³⁰: Wasserstoffatom, ein Halogenatom oder eine (C₁ bis C₄)-Alkylgruppe,
mit der Maßgabe, dass G²⁹ in meta-Position oder ortho-Position zum Strukturfragment NG²⁸ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für (C₁ bis C₄)-Alkylreste sind die Gruppen -CH₃,-CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₃ bis C₆)-Cycloalkylgruppen sind die Cyclopropyl, die Cyclopentyl und die Cyclohexylgruppe.
Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃,-OC(CH₃)₃, insbesondere eine Methoxy- oder eine Ethoxygruppe.
Weiterhin können als bevorzugte Beispiele für eine (C₁ bis C₄)-Monohydroxyalkylgruppe -CH₂OH, -CH₂CH₂OH, -CH₂CH₂CH₂OH, -CH₂CH(OH)CH₃, -CH₂CH₂CH₂CH₂OH genannt werden, wobei die Gruppe -CH₂CH₂OH bevorzugt ist. Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und -NH₃⁺.
Beispiele für (C₁ bis C₄)-Monoalkylaminogruppen sind -NHCH₃, -NHCH₂CH₃, -NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃)₂, -CH₂CH₂CH₂-O-CH(CH₃)₂. Beispiele für (C₁ bis C₄)-Alkoxy-(C₁ bis C₄)-alkoxygruppen sind die Gruppen -O-CH₂CH₂-O-CH₃, -O-CH₂CH₂CH₂-O-CH₃, -O-CH₂CH₂-O-CH₂CH₃, -O-CH₂CH₂CH₂-O-CH₂CH₃, -O-CH₂CH₂-O-CH(CH₃)₂, -O-CH₂CH₂CH₂-O-CH(CH₃)₂.
Beispiele für Hydroxy-(C₁ bis C₄)-alkoxyreste sind -O-CH₂OH, -O-CH₂CH₂OH, -O-CH₂CH₂CH₂OH, -O-CH₂CH(OH)CH₃, -O-CH₂CH₂CH₂CH₂OH.

Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Ein Beispiel für Arylgruppen ist die Phenylgruppe, die auch substituiert sein kann.
Beispiele für Aryl-(C₁ bis C₄)-alkylgruppen sind die Benzylgruppe und die 2-Phenylethylgruppe.

Bevorzugte direktziehende Farbstoffe, die in den erfindungsgemäßen Mitteln als farbgebende Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

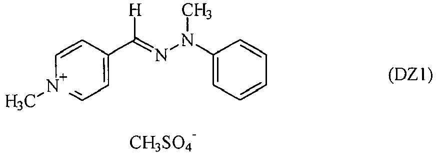

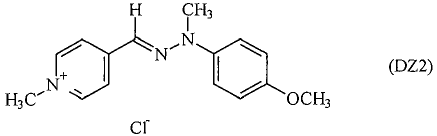

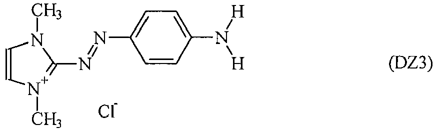

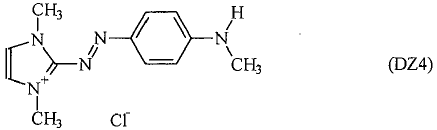

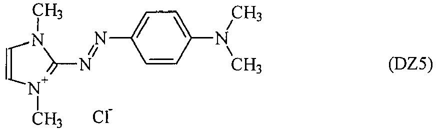

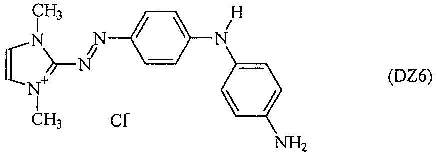

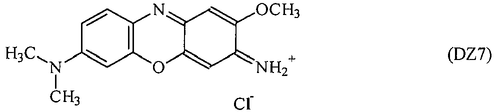

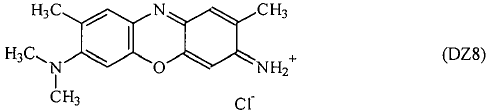

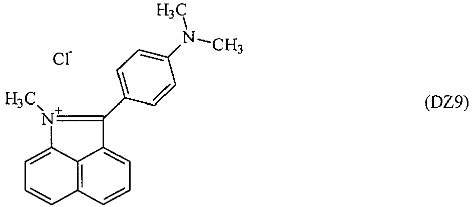

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel enthalten die direktziehenden Farbstoffe bevorzugt in einer Menge von 0,01 bis 20 Gew.-%, bezogen auf das anwendungsbereite Mittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Das erfindungsgemäße Mittel kann die eigentliche oxidative Färbung der Fasern grundsätzlich mit Luftsauerstoff ausbilden. Bevorzugt wird jedoch ein zusätzliches Oxidationsmittel eingesetzt. Wenn neben der Färbung mit den farbgebenden Komponenten ein Aufhelleffekt an menschlichem Haar gewünscht ist, wird generell ein zusätzliches Oxidationsmittel verwendet. Die Oxidationsmittelzusammensetzung umfasst einen kosmetischen Träger und mindestens ein Oxidationsmittel. Im Sinne der Erfindung sind die Oxidationsmittel von Luftsauerstoff verschieden und besitzen ein solches Oxidationspotenzial, das es ermöglicht das natürliche Farbpigment Melanin oxidativ aufzuhellen und/oder ein Oxidationsfarbstoffvorprodukt vom Entwicklertyp zu oxidieren.
Als Oxidationsmittel kommen Persulfate, Chlorite, organische Percarbonsäuren, organische Peroxide und insbesondere Wasserstoffperoxid oder dessen Anlagerungsprodukte an Harnstoff, Melamin sowie an Natriumborat in Frage.

Als organische Percarbonsäuren eignen sich erfindungsgemäß besonders Monoperphthalsäure, Peressigsäure oder 6-(Phthalimidoperoxy)hexansäure (CAS-Nr.: 128275-31-0).

Als Oxidationsmittel kommt bevorzugt Wasserstoffperoxid und/oder mindestens ein Anlagerungsprodukt davon, insbesondere an anorganische oder organische Verbindungen, in Frage. Die geeigneten Anlagerungsprodukte von Wasserstoffperoxid werden wiederum bevorzugt ausgewählt aus mindestens einer Verbindung der Gruppe, bestehend aus Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid.

Erfindungsgemäß können die Oxidationsmittel auch zusammen mit einem Katalysator Anwendung finden. Der Katalysator aktiviert die Oxidation des Substrats, wie beispielsweise die Oxidation der Oxidationsfarbstoffvorprodukte oder des Melanins. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann die Oxidation beschleunigt als auch im Rahmen einer oxidativen Färbung die Farbnuance gezielt beeinflusst werden.

Geeignete Enzyme sind z. B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation von Farbstoffvorprodukten sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das Oxidationsmittel ist bevorzugt in einer Menge von 1,0 bis 15 Gew.-%, insbesondere von 3,0 bis 12,0 Gew.-%, jeweils bezogen auf das Gewicht des Mittels, in einem erfindungsgemäßen Mittel enthalten.

Die erfindungsgemäßen Effekte können in synergistischer Weise gesteigert werden, wenn das erfindungsgemäße Mittel zusätzlich
(c) mindestens einen Wirkstoff enthält, der ausgewählt wird, aus mindestens einem Vertreter der Gruppe, die gebildet wird, aus
   c1) Proteinhydrolysaten,
   c2) Silikonderivaten,
   c3) Polyphenolen und
   c4) (Pseudo)Ceramide.

Die Proteinhydrolysate c1) sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Keratin DEC® (Vincience), Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

Bevorzugt sind die Proteinhydrolysate in einer Menge von 0,05 bis 5 Gew.-%, besonders bevorzugt von 0,5 bis 2,0 Gew.-%, jeweils bezogen auf das Gewicht des anwendungsbereiten Mittels, enthalten.

Die Silikonderivate c2) sind, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone c2) ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Besonders bevorzugte erfindungsgemäße kosmetische oder dermatologische Zubereitungen sind dadurch gekennzeichnet, dass sie mindestens ein Silikon der Formel (Si-1)

(CH₃)₃Si-[O-Si(CH₃)₂]ₓ-O-Si(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten kosmetischen oder dermatologischen Zubereitungen enthalten ein Silikon der vorstehenden Formel (Si-1). Diese Silikone werden nach der INCI-Nomenklatur als Dimethicone bezeichnet. Es werden im Rahmen der vorliegenden Erfindung als Silikon der Formel (Si-1) vorzugsweise die Verbindungen:
(CH₃)₃Si-O-Si(CH₃)₃
(CH₃)₃Si-O-(CH₃)₂Si-O-Si(CH₃)₃
(CH₃)₃Si-[O-(CH₃)₂Si]₂-O-Si(CH₃)₃
(CH₃)₃Si-[O-(CH₃)₂Si]₃-O-Si(CH₃)₃
(CH₃)₃Si-[O-(CH₃)₂Si]₄-O-Si(CH₃)₃
(CH₃)₃Si-[O-(CH₃)₂Si]₅-O-Si(CH₃)₃
(CH₃)₃Si-[O-(CH₃)₂Si]₆-O-Si(CH₃)₃
(CH₃)₃Si-[O-(CH₃)₂Si]₇-O-Si(CH₃)₃
(CH₃)₃Si-[O-(CH₃)₂Si]₈-O-Si(CH₃)₃
(CH₃)₃Si-[O-(CH₃)₂Si]₉-O-Si(CH₃)₃
(CH₃)₃Si-[O-(CH₃)₂Si]₁₀-O-Si(CH₃)₃
(CH₃)₃Si-[O-(CH₃)₂Si]₁₁-O-Si(CH₃)₃
(CH₃)₃Si-[O-(CH₃)₂Si]₁₂-O-Si(CH₃)₃
(CH₃)₃Si-[O-(CH₃)₂Si]₁₃-O-Si(CH₃)₃
(CH₃)₃Si-[O-(CH₃)₂Si]₁₄-O-Si(CH₃)₃
(CH₃)₃Si-[O-(CH₃)₂Si]₁₅-O-Si(CH₃)₃
(CH₃)₃Si-[O-(CH₃)₂Si]₁₆-O-Si(CH₃)₃
(CH₃)₃Si-[O-(CH₃)₂Si]₁₇-O-Si(CH₃)₃
(CH₃)₃Si-[O-(CH₃)₂Si]₁₈-O-Si(CH₃)₃
(CH₃)₃Si-[O-(CH₃)₂Si]₁₉-O-Si(CH₃)₃
(CH₃)₃Si-[O-(CH₃)₂Si]₂₀-O-Si(CH₃)₃
eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]2-O-Si(CH₃)₃ besonders bevorzugt sind.

Selbstverständlich können auch Mischungen der o.g. Silikone in den bevorzugten erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})ₓ(R_{c}SiO_{(4-c)/2})_{y}M (Si-2)

Beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist, worin

- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-,-OCH₂CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH₂NH₂-Rest. Eine andere mögliche Formel für besagtes Z ist -N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO_{(4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte erfindungsgemäße kosmetische oder dermatologische Zubereitungen enthalten ein aminofunktionelles Silikon der Formel (Si-3)

R'ₐG₃₋ₐ-Si(OSiG₂)ₙ-(OSiG_{b}R'_{2-b})ₘ-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus

-Q-N(R")-CH₂-CH₂-N(R")₂
-Q-N(R")₂
-Q-N⁺(R")₃A⁻
-Q-N⁺H(R")₂A⁻
-Q-N⁺H₂(R")A⁻
-Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichung: Aminopropyl Phenyl Trimethicone), DC 5-7113 (Hersteller Dow Corning, INCI-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Besonders bevorzugte erfindungsgemäße Mittel sind dadurch gekennzeichnet, dass sie mindestens es ein aminofunktionelles Silikon der Formel (Si3-a) 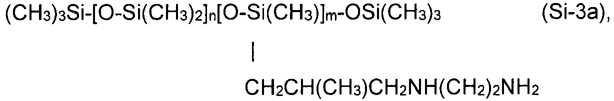 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 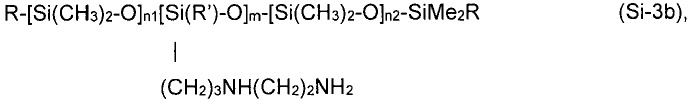 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silikone eingesetzt werden, sind erfindungsgemäße kosmetische oder dermatologische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 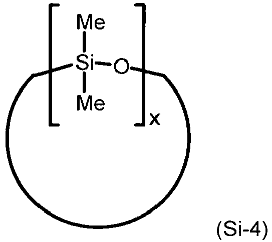 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfasst dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Si-haltige Gruppen, O-haltige Gruppen usw.. Vorzugsweise sind mindestens 80% der Reste R Alkylreste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfasst, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist.

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderung bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethyltinn-dineodecanoat, Dibutylzinn-dimethoxid, Isobutylzinn-triceroat, Dimethylzinndibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titan-naphthanat, Ethyltriethanolamin-Titanat, Titani-diisopropyl-diethyl-acetoacetat, Titan-diisopropoxy-diacetyl-acetonat und Titani-tetra-Alkoxide, bei denen das Alkoxid Butoxy oder Propoxy ist.

Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel der Ausführungsform mit einem Silikon c2) sind dadurch gekennzeichnet, dass das Silikon c2) wasserlöslich ist.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 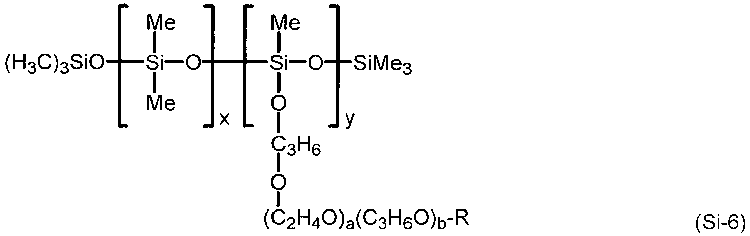 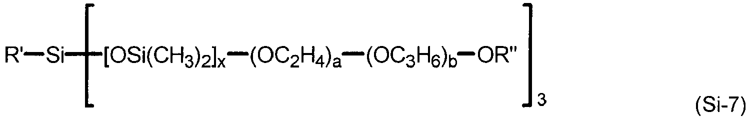 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Verbindungen die unter die oben genannten Formeln fallen, werden in den folgenden Patentanmeldungen, auf die explizit Bezug genommen wird, offenbart: US-A-4,122,029; US-A-4,265,878; US-A-4,421,769 und GB-A-2,066,659.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193 (Dow).

Polyphenole c3) sind allgemein Verbindungen, die mehr als zwei Phenol- (Polyol-) oder Phenolethergruppen enthalten, die unterschiedlichen Stoffklassen angehören. Die Polyphenole werden bevorzugt ausgewählt aus mindestens einem Vertreter der Gruppe, die gebildet wird, aus:
c3-1) Hydroxyzimtsäuren,
c3-2) 6,7-Dihydroxycumarine,
c3-3) Hydroxybenzoesäuren,
c3-4) Catechine,
c3-5) Leukoanthocyanidine,
c3-6) Anthocyanidine,
c3-7) Flavanone und
c3-8) Flavone und
c3-9) Flavonole.

In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho und Eichenrinde sowie anderer Baumrinden, Blättern von Grünem Tee (camellia sinensis) und Mate. Ebenfalls besonders bevorzugt sind die Tannine.

Als Ceramide c4) finden bevorzugt die Sphingolipide der Formel (CE-I) Verwendung, 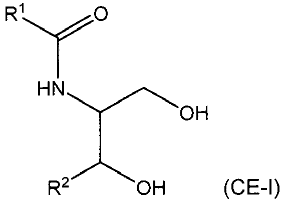 worin
- R¹: steht für eine lineare C₁₂- bis C₃₀-Alkylgruppe oder eine gesättigte oder ungesättigte ω-(C₁₂-bis C₂₀)-Acyloxy-(C₁₂-C₃₀)-alkylgruppe (wie bevorzugt Tricosanyl, Heptadecanyl oder w-(Octadeca-9,12-dien-oyloxy)-nonacosanyl) und
- R²: steht für eine gesättigte oder ungesättigte C₁₅-Alkylgruppe oder eine gesättigte oder ungesättigte C₁₅-Hydroxyalkylgruppe (wie beispielsweise Pentadeca-1-en-1-yl, 3-Hydroxypentadeca-1-en-1-yl, 1-Hydroxypentadecanyl).

Als bevorzugte Verbindungen gemäß Formel VI werden erfindungsgemäß die unter den INCI-Bezeichnungen bekannten Verbindungen Ceramide I, Ceramide II, Ceramide 1, Ceramide 2, Ceramide 3, Ceramide 5 und Ceramide 6 als Wirkstoff (W) verwendet. Besonders bevorzugt werden Gemische der Verbindungen mit der Formel (CE-I) verwendet, die beispielsweise unter dem Handelsnamen SK-Influx^{®} und Ceramide III jeweils von der Firma Degussa Care Specialties erhältlich sind, sowie das Handelsprodukt Ceramide TIC-001, das von der Firma Takasago International Corporation vermarktet wird. Die Verbindungen der Formel (CE-I) werden bevorzugt in einer Menge von 0,01 bis 1,0 Gew.-% bezogen auf das Gewicht des anwendungsbereiten kosmetischen Mittels verwendet.

Außerdem sind Pseodoceramide, wie insbesondere das Pseudoceramid N-(C₈₋₂₂-Acyl)-C₈₋₂₂-acyl-hydroxyprolin (wie beispielsweise das Cetyl-hydroxyprolin Palmitamide gemäß Handelsprodukt Sym Repair 153884 der Firma Symrise) erfindungsgemäße Pseudoceramide c4).

Das gebrauchsfertige erfindungsgemäße Mittel sollte bevorzugt einen pH-Wert im Bereich von pH 5 bis pH 12, insbesondere von pH 7 bis pH 11, aufweisen. Besonders bevorzugt ist die Anwendung der Haarfärbemittel in einem schwach alkalischen Milieu in einem pH-Bereich zwischen pH 7 und pH 9.

Die erfindungsgemäßen Mittel können zusätzlich alle auf diesen Gebieten bekannten und üblicherweise verwendeten Wirk- und Hilfsstoffe enthalten.

Die eingesetzten Verbindungen der Formel (I) können die in Farbveränderungsmitteln üblicherweise verwendeten anionischen Tenside ganz oder teilweise ersetzen. Damit können die Verbindungen der Formel (I) als alleiniges anionisches Tensid in den Mitteln eingesetzt werden, oder es können Gemische mit üblichen weiteren anionischen Tensiden eingesetzt werden. Diese weiteren anionischen Tenside sind an späterer Stelle näher erläutert. Beispielsweise können die Verbindungen der Formeln (I) und weitere anionische Tenside in einem Gewichtsverhältnis im Bereich von 1 : 0,002 bis 3,5 : 1, besonders bevorzugt 1 : 0,5 bis 1 : 2, insbesondere 1 : 0,75 bis 1 : 1,5 vorliegen.

Insbesondere ist es, wie zuvor erwähnt, erfindungsgemäß bevorzugt, Alkylsulfate bzw. Alkylethersulfate, speziell Laurethsulfat ganz oder teilweise durch die erfindungsgemäß eingesetzten Verbindungen der Formel (I) zu ersetzen. In einer bevorzugten Ausführungsform enthält das erfindungsgemäße, insbesondere ammoniakarme, Mittel als limitierten Gehalt dieser Tenside daher 0 bis 0,5 Gew.-%, insbesondere 0 bis 0,3 Gew.-% mindestens eines anionischen Tensids, ausgewählt aus
- linearen oder verzweigten C₈ bis C₄₀-Alkylsulfaten,
- linearen oder verzweigten C₈ bis C₄₀-Alkylethersulfaten mit mindestens einer Ethylenoxid-Einheit oder Propylenoxid-Einheit im Molekül.

In Mitteln zur oxidativen Färbung von Haaren wird insbesondere beim Ersetzen eines Teils oder des ganzen herkömmlichen anionischen Tensids, wie Alkylsulfate bzw. Alkylethersulfate, insbesondere Laurethsulfate, durch die erfindungsgemäß eingesetzten Verbindungen der Formeln (I) bei der Ausfärbung und nachfolgend Untersuchung von Colorationen eine deutlich erhöhte Farbstabilität und damit Farbechtheit festgestellt.

In einer weiteren Ausführungsform können die erfindungsgemäßen Mittel mindestens ein zusätzliches Tensid enthalten, welches von Verbindungen der Formeln (I) verschieden ist. Prinzipiell eignen sich sowohl weitere anionische als auch zwitterionische, ampholytische, nichtionische und kationische Tenside. In vielen Fällen hat es sich aber als vorteilhaft erwiesen, die Tenside aus anionischen, zwitterionischen oder nichtionischen Tensiden auszuwählen.

Als weitere anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, -phosphaten und/oder - isethionaten, die sich von Alkyl- und/oder Alkenyloligoglykosiden der allgemeinen Formel (II) ableiten,

   R-O-(G)ₚ (II)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10,
   insbesondere das Laurylglucosidcarboxylat, wie es als Plantapon^{®} LGC von Cognis Deutschland erhältlich ist,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte zusätzliche anionische Tenside werden ausgewählt aus oben genannten anionischen Alkyloligoglykosid- bzw. anionischen Alkenyloligoglykosid-Derivaten, Ethercarbonsäuren mit 10 bis 18 C-Atomen in der Alkylgruppe und bis zu 12 Glykolethergruppen im Molekül sowie insbesondere Salzen von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Das erfindungsgemäße Mittel enthält die erfindungsgemäßen Inhaltsstoffe in einem Träger. Solche Träger sind beispielsweise Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die Träger können insbesondere wässrig oder wässrigalkoholisch sein.

Ein wässriger Träger enthält mindestens 50 Gew.-% Wasser.

Unter wässrig-alkoholischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Ein weiterer Gegenstand der vorliegenden Erfindung ist ein Verfahren zur Behandlung keratinhaltiger Fasern, insbesondere menschlicher Haare, bei dem ein Mittel des ersten Erfindungsgegenstandes auf die Fasern aufgetragen wird und nach einer Einwirkzeit wieder abgespült wird.

Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Im ersten Erfindungsgegenstand beschriebene Mittel eignen sich besonders gut zur erfindungsgemäßen Verwendung auf keratinhaltigen Fasern, insbesondere menschlichen Haaren.

Unter keratinhaltigen Fasern sind Wolle, Pelze, Federn und insbesondere menschliche Haare zu verstehen. Die erfindungsgemäße Kombination kann prinzipiell aber auch auf anderen Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Ein dritter Gegenstand der Erfindung ist daher die Verwendung eines, insbesondere ammoniakfreien, Mittels zur Färbung keratinhaltiger Fasern, insbesondere menschlicher Haare, welches
(i) 2-(2-Hydroxyethyl)-p-phenylendiamin oder eines seiner physiologisch verträglichen Salze,
(ii) mindestens eine Verbindung aus den N-acylierten Aminosäurederivaten der Formel (I) 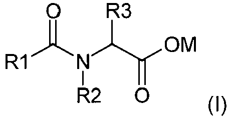 worin
   R¹ eine (C₇ bis C₃₀)-Alkylgruppe, (C₇ bis C₃₀)-Alkenylgruppe oder eine
   (C₇ bis C₃₀)-Hydroxyalkylgruppe bedeutet,
   R² und R³ voneinander unabhängig für ein Wasserstoffatom, eine (C₁ bis C₄)-Alkylgruppe oder eine (C₁ bis C₄)-Hydroxyalkylgruppe steht,
   und
   M ein Wasserstoffatom oder ein Äquivalent eines ein- oder mehrwertigen Kations bedeutet,
(iii) sowie mindestens ein Hydroxyalkylamin
enthält.

Für die bevorzugten Mittel gelten mutatis mutandis die entsprechenden Ausführungsformen des ersten Erfindungsgegenstandes.

Bevorzugt werden die erfindungsgemäßen Mittel
(i) zur Steigerung der Grauabdeckung
(ii) zur Verringerung der Farbmittelpenetration in die Haut
verwendet.

Das erfindungsgemäße Mittel kann in einer Verpackungseinheit (Kit), welche mindestens ein (insbesondere getrennt konfektioniertes) erfindungsgemäßes Mittel des ersten Erfindungsgegenstandes enthält, bereitgestellt werden. Zusätzlich kann das Kit bevorzugt mindestens eine (insbesondere getrennt konfektionierte) Oxidationsmittelzusammensetzung enthalten. Darüber hinaus kann das Kit zusätzlich optional eine Gebrauchsanweisung, Applikationshilfen, Anmischgefäße oder Schutzhandschuhe enthalten.

Die erfindungsgemäße Lehre lässt sich in Gegenwart mindestens eines Oxidationsmittels durchführen. Bevorzugt geeignete Oxidationsmittel sind die des ersten Erfindungsgegenstandes.

Das eigentliche Haarfärbemittel wird zweckmäßigerweise unmittelbar vor der Anwendung durch Mischung der Oxidationsmittelzusammensetzung mit dem Träger der farbgebenden Komponenten, bevorzugt im Gewichtsverhältnisbereich von 1 zu 4 bis 4 zu 1, insbesondere von 1 zu 2 bis 2 zu 1, hergestellt.

### Beispiele

Es wurden folgende Zusammensetzungen der nachfolgenden Tabellen 1 und 2 nach Standardherstellverfahren bereitgestellt. Alle Mengenangaben sind - wenn nicht anders gekennzeichnet - Gewichtsprozent. Folgende Handelsprodukte wurden als Rohstoffe verwendet:

| | |
|---|---|
| Hydrenol^{®} D | C₁₆-C₁₈-Fettalkohol (INCI-Bezeichnung: Cetearyl alcohol) (Cognis Deutschland) |
| Lorol^{®} techn. | C₁₂-C₁₈-Fettalkohol (INCI-Bezeichnung: Coconut alcohol) (Cognis Deutschland) |
| Eumulgin^{®} B 1 | Cetylstearylakohol mit 12 EO-Einheiten (EO) (INCI-Bezeichnung: Ceteareth-12) (Cognis Deutschland) |
| Eumulgin^{®} B2 | Cetylstearylalkohol mit ca. 20 EO (INCI-Bezeichnung: Ceteareth-20) (Cognis Deutschland) |
| Akypo^{®} Soft 45 NV | 2-(C₁₂₋₁₄-Fettalkoholethoxylat (4.5 EO))-essigsäure Natriumsalz; 21% Aktivsubstanz in Wasser; INCI-Bezeichnung: Sodium Laureth-5 Carboxylate (KAO) |
| Protelan^{®} MST 35 | Gemisch aus Natrium N-Myristoylsarcosinate und Natrium N-Methyl-N-cocoyltaurate (40 Gew.-% Aktivsubstanz, Zschimmer & Schwartz) |
| Polymer W 37194 | ca. 20Gew.-% Aktivsubstanzgehalt in Wasser; INCI-Bezeichnung: Acrylamidopropyltrimonium Chloride/Acrylates Copolymer (Stockhausen) |
| Turpinal^{®} SL | 1-Hydroxyethan-1,1-diphosphonsäure (INCI-Bezeichnung: Etidronic Acid, Aqua (Water)) (Solutia) |
| Texapon^{®} NSO UP | Natriumlaurylethersulfat (27 % Aktivsubstanz in Wasser; INCI: Sodium Laureth Sulfate) (Hersteller: COGNIS) |
| Aculyn^{®} 33 | 30 Gew.-% Aktivsubstanz in Wasser (INCI-Bezeichnung: Acrylates Copolymer) (Rohm & Haas) |

**Tabelle 1: Färbecremes**

| | A-1 | A-2 | A-3 | A-4 |
|---|---|---|---|---|
| Hydrenol D | 7,15 | 7,15 | 7,15 | 7,15 |
| Lorol | 2,60 | 2,60 | 2,60 | 2,60 |
| Eumulgin B 2 | 0,65 | 0,65 | 0,65 | 0,65 |
| Eumulgin B 1 | 0,65 | 0,65 | 0,65 | 0,65 |
| Akypo Soft 45 NV | 10,00 | 10,00 | 10,00 | 10,00 |
| Protelan MST 35 | 6,00 | 6,00 | 6,00 | 6,00 |
| Polymer W 37194 | 3,00 | 3,00 | 3,00 | 3,00 |
| Monoethanolamin | 5,00 | 5,00 | 5,00 | 5,00 |
| Ascorbinsäure | 0,10 | 0,10 | 0,10 | 0,10 |
| Turpinal SL | 0,20 | 0,20 | 0,20 | 0,20 |
| Natronwasserglas 40/42 | 0,50 | 0,50 | 0,50 | 0,50 |
| Glycin | 1,00 | 1,00 | 1,00 | 1,00 |
| 2-(2-Hydroxyethyl)-p-phenylendiamin sulfat | 1,20 | 2,60 | 1,60 | 1,00 |
| 2,4,5,6-Tetraaminopyrimidin sulfat | 0,20 | - | - | - |
| p-Toluylendiamin sulfat | - | - | 0,90 | - |
| 2-Chlor-6-methyl-3-aminophenol | - | 0,20 | 0,20 | - |
| 2,7-Dihydroxynaphtalin | 0,90 | 1,00 | 1,00 | 0,10 |
| 2-Methylresorcin | 0,10 | - | - | 0,10 |
| 2-Amino-3-hydroxypyridin | - | - | - | 0,30 |
| 3-Amino-2-methylamino-6-methoxypyridin | - | 0,80 | 0,80 | - |
| Wasser | ad 100 | ad 100 | ad 100 | ad 100 |

**Tabelle 2: Entwickleremulsion**

| | |
|---|---|
| | |
| Dipicolinsäure | 0,10 |
| Dinatriumphosphat | 0,03 |
| Turpinal SL | 1,50 |
| Texapon NSO UP | 2,00 |
| Aculyn 33A | 12,00 |
| Wasserstoffperoxid, 50 Gew.-% | 12,00 |
| Wasser | ad 100 |

Es wurden Haarsträhnen der Fa. Kerling (80% grau, 15cm lang, ca. 2 g) verwendet. Die Strähnen wurden zunächst mit Poly Blonde Ultrablondierung für 30 min bei 32°C behandelt, mit handwarmem Wasser gespült und abschließend an der Luft getrocknet und für 24 h gelagert.

Vor der Anwendung wurde jede der Farbcremes A-1 bis A-4 der Tabelle 1 mit der Entwickleremulsion gemäß Tabelle 2 im Gewichtsverhältnis 1 zu 1 gemischt.

Die fertigen Mischungen wurden auf die vorbehandelten Strähnen gegeben und nach einer Einwirkzeit von 30 min bei 32°C abgespült. Anschließend wurden die Strähnen getrocknet.

Es wurden mit den beschriebenen Verfahren sehr gute Grauabdeckungen erzielt.

## Patentansprüche

1. Mittel zur Färbung keratinhaltiger Fasern, insbesondere menschlicher Haare, enthaltend
(i) 2-(2-Hydroxyethyl)-p-phenylendiamin und/oder mindestens eines seiner physiologisch verträglichen Salze,
(ii) ein acyliertes Aminosäurederivat der Formel (I) 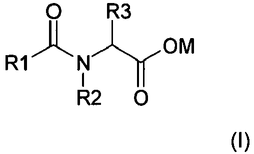 worin
R¹ eine C₇-C₃₀-Alkyl-Gruppe, eine C₇-C₃₀-Alkenyl-Gruppe oder eine C₇-C₃₀-Hydroxyalkyl-Gruppe bedeutet,
R² und R³ unabhängig voneinander für ein Wasserstoffatom, eine C₁-C₄-AlkylGruppe oder eine C₁-C₄-Hydroxyalkyl-Gruppe steht,
und
M ein Wasserstoffatom oder ein Äquivalent eines ein- oder mehrwertigen Kations bedeutet,
(iii) und mindestens ein Hydroxyalkylamin.

2. Mittel nach Anspruch 1, **dadurch gekennzeichnet, dass** das Hydroxyalkylamin aus der Gruppe ausgewählt wird, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

3. Mittel nach Anspruch 1 oder 2, **dadurch gekennzeichnet, dass** R² für ein Wasserstoffatom oder eine Methyl-Gruppe und R³ für ein Wasserstoffatom oder eine Methyl-Gruppe steht.

4. Mittel nach einem der Ansprüche 1 bis 3, **dadurch gekennzeichnet, dass** es 2-(2-Hydroxyethyl)-p-phenylendiamin und/oder dessen physiologisch verträglichen Salze in einer Menge von 0,001 bis 6 Gew.-%, bezogen auf das Gewicht des Mittels, enthält.

5. Mittel nach einem der Ansprüche 1 bis 4, **dadurch gekennzeichnet, dass** es mindestens ein Hydroxyalkylamin in einer Menge von 1 bis 8 Gew.-%, bezogen auf das Gewicht des Mittels, enthält.

6. Mittel nach einem der Ansprüche 1 bis 5, **dadurch gekennzeichnet, dass** es mindestens ein acyliertes Aminosäurederivat gemäß Formel (I) in einer Menge von 0,1 bis 10 Gew.-%, bezogen auf das Gewicht des Mittels, enthält.

7. Mittel nach einem der Ansprüche 1 bis 6, **dadurch gekennzeichnet, dass** es ammoniakfrei ist.

8. Mittel nach einem der Ansprüche 1 bis 7, enthaltend zusätzlich 0 bis 0,5 Gew.-%, insbesondere 0 bis 0,3 Gew.-%, jeweils bezogen auf das Gewicht des Färbemittels, mindestens eines anionischen Tensids, ausgewählt aus
- linearen oder verzweigten C₈ bis C₄₀-Alkylsulfaten,
- linearen oder verzweigten C₈ bis C₄₀-Alkylethersulfaten mit mindestens einer Ethylenoxid-Einheit oder Propylenoxid-Einheit im Molekül.

9. Mittel nach einem der Ansprüche 1 bis 8, **dadurch gekennzeichnet, dass** es weniger als 1 Gew.-%, bezogen auf das Gewicht des Mittels, p-Toluylendiamin oder eines seiner physiologisch verträglichen Salze enthält.

10. Verfahren zur Behandlung keratinhaltiger Fasern, bei dem ein Mittel nach einem der Ansprüche 1 bis 9 auf die Fasern aufgetragen wird und nach einer Einwirkzeit wieder abgespült wird.

11. Verwendung von einem Mittel nach einem der Ansprüche 1 bis 9 als, insbesondere ammoniakfreies, Färbemittel
(i) zur Steigerung der Grauabdeckung
(ii) zur Verringerung der Farbmittelpenetration in die Haut

12. Kit-of-Parts (Verpackungseinheit), **dadurch gekennzeichnet, dass** es mindestens ein Mittel nach einem der Ansprüche 1 bis 9 und mindestens eine Oxidationsmittelszusammensetzung umfasst.

13. Kit-of-Parts (Verpackungseinheit) nach Anspruch 12, **dadurch gekennzeichnet, dass** die Oxidationsmittelzusammensetzung als Oxidationsmittel Wasserstoffperoxid und/oder mindestens ein Anlagerungsprodukt von Wasserstoffperoxid enthält.

## Claims

1. An agent for coloring keratin-containing fibers, in particular human hair, the agent containing
(i) 2-(2-hydroxyethyl)-p-phenylenediamene and/or at least one physiologically acceptable salt thereof,
(ii) an acylated amino acid derivative of the formula (I) 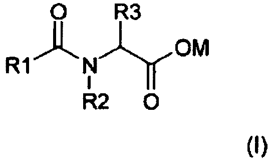 where
R¹ is a C₇-C₃₀ alkyl group, a C₇-C₃₀ alkenyl group or a C₇-C₃₀ hydroxyalkyl group,
R² and R³ independently of one another represent a hydrogen atom, a C₁-C₄ alkyl group or a C₁-C₄ hydroxyalkyl group,
and
M is a hydrogen atom or an equivalent of a monovalent or polyvalent cation,
(iii) and at least one hydroxyalkyl amine.

2. The agent according to claim 1, **characterized in that** the hydroxyalkyl amine is selected the group formed by 2-aminoethan-1-ol (monoethanolamine), 3-aminopropan-1-ol, 4-aminobutan-1-ol, 5-aminopentan-1-ol, 1-aminopropan-2-ol, 1-aminobutan-2-ol, 1-aminopentan-2-ol, 1-aminopentan-3-ol, 1-aminopentan-4-ol, 3-amino-2-methylpropan-1-ol, 1-amino-2-methylpropan-2-ol, 3-aminopropan-1,2-diol, 2-amino-2-methylpropane-1,3-diol.

3. The agent according to one of claims 1 or 2, **characterized in that** R² represents a hydrogen atom or a methyl group and R³ represents a hydrogen atom or a methyl group.

4. The agent according to one of claims 1 to 3, **characterized in that** it contains 2-(2-hydroxyethyl)-p-phenylenediamene and/or the physiologically acceptable salts thereof in an amount of from 0.001 to 6 wt.%, based on the weight of the agent.

5. The agent according to one of claims 1 to 4, **characterized in that** it contains at least one hydroxyalkyl amine in an amount of from 1 to 8 wt.%, based on the weight of the agent.

6. The agent according to one of claims 1 to 5, **characterized in that** it contains at least one acylated amino acid derivative according to formula (I) in an amount of from 0.1 to 10 wt.%, based on the weight of the agent.

7. The agent according to one of claims 1 to 6, **characterized in that** it does not contain ammonia.

8. The agent according to one of claims 1 to 7, additionally containing from 0 to 0.5 wt.%, in particular from 0 to 0.3 wt.%, based in each case on the weight of the dye, of at least one anionic surfactant, selected from
- linear or branched C₈ to C₄₀ alkyl sulfates
- linear or branched C₈ to C₄₀ alkyl ether sulfates having at least one ethylene oxide unit or propylene oxide unit in the molecule.

9. The agent according to one of claims 1 to 8, **characterized in that** it contains less than 1 wt.% p-toluylenediamene or a physiologically acceptable salt thereof, based on the weight of the agent.

10. A method for treating keratin-containing fibers, in which an agent according to one of claims 1 to 9 is applied to the fibers and is rinsed off again after a reaction time.

11. The use of an agent according to one of claims 1 to 9 as an in particular ammonia-free dye
(i) for increasing the coverage of grey hair
(ii) for reducing the degree to which the dye penetrates the skin.

12. A kit-of-parts (packaging unit), **characterized in that** it contains at least one agent according to one of claims 1 to 9 and at least one oxidizing agent composition.

13. The kit-of-parts (packaging unit) according to claim 12, **characterized in that** the oxidizing agent composition contains hydrogen peroxide and/or at least one product of the addition of hydrogen peroxide as the oxidizing agent.

## Revendications

1. Produit de coloration de fibres contenant de la kératine, en particulier de cheveux humains, contenant :
i) de la 2-(2-hydroxyméthyl)paraphénylène diamine et/ou au moins un de ses sels physiologiquement acceptables,
ii) un dérivé d'acide aminé acylé de formule (I) : 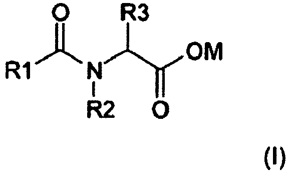 dans lequel
R¹ représente un groupe C₇₋₃₀-alkyle, un groupe C₇₋₃₀-alcényle ou un groupe C₇₋₃₀-hydroxyalkyle,
R² et R³ représentent indépendamment l'un de l'autre un atome d'hydrogène, un groupe C₁₋₄-alkyle ou un groupe C₁₋₄hydroxyalkyle, et
M représente un atome d'hydrogène ou un équivalent d'un cation monovalent ou polyvalent, te
iii) au moins une hydroxyalkylamine.

2. Produit selon la revendication 1, **caractérisé en ce que** l'hydroxyalkylamine est choisie dans le groupe formé du 2-aminoéthan-1-ol (monoéthanolamine), 3-aminopropan-1-ol, 4-aminobutan-1-ol, 5-aminopentan-1-ol, 1-aminopropan-2-ol, 1-aminobutan-2-ol, 1-aminopentan-2-ol, 1-aminopentan-3-ol, 1-aminopentan-4-ol, 3-amino-2-méthylpropan-1-ol, 1-amino-2-méthylpropan-2-ol, 3-aminopropan-1,2-diol, 2-amino-2-méthylpropan-1,3-diol.

3. Produit selon une des revendications 1 ou 2, **caractérisé en ce que** R² représente un atome d'hydrogène ou un groupe méthyle et R³ représente un atome d'hydrogène ou un groupe méthyle.

4. Produit selon une des revendications 1 à 3, **caractérisé en ce qu'**il contient de la 2-(2-hydroxyméthyl)paraphénylène diamine et/ou au moins un de ses sels physiologiquement acceptables à hauteur de 0,001 à 6 % en poids rapporté au poids du produit.

5. Produit selon une des revendications 1 à 4, **caractérisé en ce qu'**il contient au moins une hydroxyalkylamine à hauteur de 1 à 8 % en poids rapporté au poids du produit.

6. Produit selon une des revendications 1 à 5, **caractérisé en ce qu'**il contient au moins un dérivé d'acide aminé acylé de formule (I) à hauteur de 0,1 à 10 % en poids rapporté au poids du produit.

7. Produit selon une des revendications 1 à 6, **caractérisé en ce qu'**il ne contient pas d'ammoniac.

8. Produit selon une des revendications 1 à 7, contenant en plus 0 à 0,5 % en poids, en particulier 0 à 0,3 % en poids, rapporté au poids du produit colorant, au moins un tensioactif anionique choisi parmi :
i. des sulfates de C₈₋₄₀-alkyle linéaires ou ramifiés, choisis parmi :
ii. des sulfates de C₈₋₄₀-alkyléthers linéaires ou ramifiés avec au moins une unité oxyde d'éthylène ou oxyde de propylène dans la molécule.

9. Produit selon une des revendications 1 à 8, **caractérisé en ce qu'**il contient moins de 1 % en poids, rapporté au poids du produit, de paratoluyène diamine ou un de ses sels physiologiquement acceptables

10. Procédé de traitement de fibres contenant de la kératine, dans lequel un produit selon une des revendications 1 à 9 est appliqué sur les fibres et rincé après une durée d'action.

11. Utilisation d'un produit selon une des revendications 1 à 9, en particulier d'un colorant sans ammoniac :
i) pour augmenter la couverture des cheveux gris
ii) pour réduire la pénétration du colorant dans la peau.

12. Kit (unité d'emballage) **caractérisé en ce qu'**il comprend au moins un produit selon une des revendications 1 à 9 et au moins une composition oxydante.

13. Kit (unité d'emballage) selon la revendication 12, **caractérisé en ce que** la composition oxydante contient comme oxydant du peroxyde d'hydrogène et/ou au moins un produit pour déposer le peroxyde d'hydrogène.
